# EUROPÄISCHE PATENTANMELDUNG

(11) **EP 3 644 085 A1**
(43) Veröffentlichungstag der Anmeldung: **29.04.2020**
(21) Anmeldenummer: 18202596.5
(22) Anmeldetag: 25.10.2018
(51) Int. Cl.: G01R 33/36, G01R 33/54

(54) **AUSWAHL VON MESSSPULEN BEI DER MAGNETRESONANZ-BILDGEBUNG**

(71) Anmelder: Siemens Healthcare GmbH, 91052 Erlangen (DE)
(72) Erfinder: Meyer, Stefan, 91094 Langensendelbach (DE); Rick, Manuela, 91080 Uttenreuth (DE)

(57) **Zusammenfassung**

Die Erfindung betrifft ein Verfahren und eine Magnetresonanzanlage mit Techniken zur Auswahl von ein oder mehreren Messspulen bei der Magnetresonanz-Bildgebung. Dabei kann eine Vorauswahl bei der Auswahl der ein oder mehreren Messspulen aus einer Vielzahl von Kandidaten-Spulen berücksichtigt werden.

## Beschreibung

### TECHNISCHES GEBIET

Verschiedene Beispiele der Erfindung betreffen Techniken, um ein oder mehrere Messspulen bei der Magnetresonanz-Bildgebung auszuwählen.

### HINTERGRUND

Bei der Magnetresonanz(MR)-Bildgebung können unterschiedliche anatomische Regionen einer Untersuchungsperson untersucht werden. Dazu können Kernspins in einem Untersuchungsbereich durch Einstrahlen von Hochfrequenz(HF)-Pulsen aus der Ruhelage ausgelenkt werden. Anschließend können Rohdaten erfasst werden, welche HF-Signalen der relaxierenden Kernspins entsprechen. Daraus können ein oder mehrere MR-Bilder erzeugt werden. Zum Einstrahlen und Empfangen von HF-Signalen werden Messspulen verwendet.

Der Betrieb von MR-Anlagen erfordert typischerweise das Einstellen einer großen Anzahl von Betriebsparametern. Beispielsweise müssen ein oder mehrere MR-Protokolle ausgewählt werden, welche die Abfolge von eingestrahlten HF-Pulse und Zeitdauern zum Empfangen von HF-Signalen sowie weitere Parameter, wie beispielsweise Gradientenpulse, definieren. Typischerweise korreliert die Auswahl des geeigneten MR-Protokolls stark mit der Auswahl des geeigneten Messbereichs bzw. der Auswahl der passenden Messspule. Wird beispielsweise die "falsche" oder überflüssige Messspule ausgewählt, so wird die Qualität der MR-Bilder herabgesetzt sein, bzw. es kann erforderlich sein, oder die Messung zu wiederholen

In Referenzimplementierungen werden automatisch alle Messspulen ausgewählt, die im Messbereich (auch als Untersuchungsvolumen bezeichnet) liegen. Dies schränkt die Einsatzmöglichkeiten der automatischen Spulenselektion ein. Beispielsweise wird bei einer Knieuntersuchung die Kniespule auf der fest im Tisch installierten Wirbelsäulenspule positioniert. Eine automatische Anwahl beider Spulen würde jedoch vermehrt zu Einfaltungsartefakten führen, weshalb nur die automatische Auswahl der Kniespule gewünscht ist. Daraus resultiert häufig, dass eine Vielzahl von MR-Protokollen in einer Protokoll-Datenbank abgelegt werden, wobei die verschiedenen MR-Protokolle dann mit unterschiedlichen Messspulen fix assoziiert sind, d.h. keine automatische Spulenselektion genutzt wird. Dadurch beobachtet man, dass manchmal die falsche Messspule verwendet wird, so dass die MR-Bilder eine herabgesetzte Qualität aufweisen, oder aber die Messzeitdauer stark erhöht ist, bzw. wiederholt werden müssen. Beispielsweise kann es vorkommen, dass sich in der Messphase (d.h., zur Laufzeit unmittelbar vor oder bei Durchführung der MR-Bildgebung, wenn der Patient bereits in der MR-Anlage befindlich ist) je nach Patientenlagerung, bzw. Patientengröße neue Anforderungen an die Auswahl an die Messspule ergeben. Dies kann in Referenzimplementierungen - d.h. bei fest mit ein oder mehreren Messspulen assoziierten MR-Protokollen - nicht oder nur schwer zu berücksichtigen sein. D.h., es kommt gegebenenfalls zu einer suboptimalen Auswahl der Messspule mit eingeschränkter Bildqualität. Dies gilt auch für einen manuellen Modus, bei welchem der Nutzer einzelne Spulen aus allen verfügbaren Kandidaten-Spulen aktivieren oder deaktivieren kann. Um den Arbeitsablauf am Scanner einfach zu halten, wird die Spulenselktion im manuellen Modus typischerweise während einer Planungsphase (d.h. vor der Messphase) ausgeführt, so dass eine Anpassung während der Messphase - z.B. an die konkrete Patientenlagerung oder -größe - nicht notwendig ist.
Dies berücksichtigt aber dann nicht eventuelle Besonderheiten durch Verwendung einer anderen Spule, einer anderen Lagerung, Änderungen durch die Patientenphysiognomie, ets. Außerdem ist das Durchführen einer manuellen Auswahl von Spulen zeitaufwändig und fehleranfällig, so dass die Verfügbarkeit einer MR-Anlage leidet.

### KURZE BESCHREIBUNG DER ERFINDUNG

Deshalb besteht ein Bedarf für verbesserte Techniken zur Auswahl von mindestens einer Messspule aus einer Vielzahl von Kandidaten-Spulen einer MR-Anlage bei der MR-Bildgebung. Insbesondere besteht ein Bedarf für Techniken, welche zumindest einige der vorgenannten Nachteile und Einschränkungen beheben.

Diese Aufgabe wird gelöst von den Merkmalen der unabhängigen Patentansprüche. Die Merkmale der abhängigen Patentansprüche definieren Ausführungsformen.

In einem Beispiel umfasst ein Verfahren zur Auswahl von mindestens einer Messspule aus einer Vielzahl von Kandidaten-Spulen einer MR-Anlage bei der MR-Bildgebung das Empfangen von ersten Steuerdaten. Die ersten Steuerdaten indizieren einen Messbereich. Außerdem umfasst das Verfahren das Empfangen von zweiten Steuerdaten, welche eine Vorauswahl aus der Vielzahl von Kandidaten-Spulen indizieren. Das Verfahren umfasst ferner das Auswählen der mindestens einen Messspule aus der Vielzahl von Kandidaten-Spulen basierend auf dem Messbereich und der Vorauswahl. Ferner umfasst das Verfahren das Auslösen eines MR-Protokolls zur MR-Bildgebung mit der mindestens einen Messspule.

Beispielsweise wäre es möglich, dass die mindestens eine Messspule eine Empfangsspule umfasst, oder aber auch eine kombinierte Sende/Empfangsspule. Die Vielzahl von Kandidaten-Spulen kann in einer Spulen-Datenbank, die mit der MR-Protokollen assoziiert sind, hinterlegt sein. Die Kandidaten-Spulen können eine Gruppe von möglichen Messspulen bezeichnen, die grundsätzlich mit der MR-Anlage verwendet werden können.

Dabei kann es eine Zuordnung zwischen den verschiedenen Kandidaten-Spulen und der Positionierung auf dem Patienten geben. Zum Beispiel können unterschiedliche Kandidaten-Spulen eingerichtet sein, um in Bezug auf unterschiedliche Messbereiche positioniert zu werden. Manche der Spulen können beispielsweise in feste Bauteile der MR-Anlage, z. B. einen Tisch, etc. eingebettet sein. Andere Spulen können hingegen in beweglichen Spulenbauteilen angebracht sein, die z.B. an- und abgesteckt werden können, bzw. unterschiedlich auf dem Patienten positioniert werden können. Dabei kann ein Spulenbauteil auch mehrere Spulenelemente beinhalten.

Beispielsweise wäre es möglich, dass die ersten Steuerdaten und/oder die zweiten Steuerdaten von einer Benutzerschnittstelle der MR-Anlage empfangen werden. Beispielsweise könnte der Benutzer der MR-Anlage beim Einstellen der verschiedenen Messparameter in einer Planungsphase oder auch kurz vor der Messphase den Messbereich spezifizieren und/oder die Vorauswahl aus der Vielzahl von Kandidaten-Spulen spezifizieren.

Beispielsweise kann der Messbereich eine anatomische Region oder ein Raumvolumen spezifizieren.

Im Allgemeinen könnte die Vorauswahl positiv oder negativ definiert sein. Dies bedeutet, dass die Vorauswahl zum Beispiel ein oder mehrere Kandidaten-Spulen aus der Vielzahl von Kandidaten-Spulen ausschließen kann, so dass diese nicht bei der Auswahl berücksichtigt werden. Dies bedeutet in anderen Worten, dass ein oder mehrere nicht-selektierbare Spulen im Zusammenhang mit der Vorauswahl indiziert werden können. Andererseits wäre es aber auch möglich, dass mittels der Vorauswahl eine Präferenz für die Auswahl der Spulen implementiert wird. Beispielsweise könnte die Vorauswahl eine Teilmenge der Vielzahl von Kandidaten-Spulen definieren, welche im Zusammenhang mit Auswahl der mindestens einen Messspule selektierbar sind. Insbesondere wäre es beispielsweise möglich, dass die zweiten Steuerdaten ein oder mehrere selektierbare Spulenklassen oder Spulenkompartments umfassen. Dies bedeutet, dass die im Zusammenhang mit der Auswahl der mindestens einen Messspule die selektierbaren Spulen nicht notwendigerweise explizit durch die zweiten Steuerdaten indiziert werden müssen, sondern vielmehr auf parametrisierte Art und Weise durch die zweiten Steuerdaten indiziert werden können. Dies bedeutet in anderen Worten, dass es möglich ist, bestimmte Regeln für das Auswählen der mindestens einen Messspule mittels der Spulenklasse(n) oder Spulenkompartments(s) zu definieren.

Dabei bezeichnen Spulenkompartments typischerweise ein oder mehrere Spulenelemente eines Spulenbauteils, welches mehrere Spulenelemente umfasst. Ein Beispiel wäre zum Beispiel ein Spulenbauteil für die Bildgebung eines Untersuchungsbereichs, der dem Kopf einer Untersuchungsperson entspricht. Dabei können unterschiedliche Spulenkompartments beispielsweise unterschiedliche Positionen am Kopf, etwa vorne oder hinten, bezeichnen.

Beispielsweise wäre es möglich, dass die ein oder mehreren selektierbaren Spulenklassen oder Spulenkompartments im Zusammenhang mit einer assoziierten anatomischen Region definiert sind. Dies bedeutet, dass durch die zweiten Steuerdaten festgelegt werden kann, dass die Vorauswahl solche Spulen zulässt, welche eine Bildgebung der entsprechenden anatomischen Region ermöglichen, bzw. welche durch ein entsprechendes Spulenbauteil nahe bei der entsprechenden anatomischen Region positionierbar sind.

Derart kann insbesondere für einen Fall, dass der Messbereich um Artefakte zu verringern vergleichsweise groß ist, eine bessere Auswahl der mindestens einen Messspule erfolgen. Beispielsweise könnte der Messbereich im Zusammenhang mit einer vergleichsweisen großen anatomischen Region sein, während die ein oder mehreren selektierbaren Spulenklassen oder Spulenkompartments in Bezug auf eine entsprechende kleinere anatomische Region, die im Messbereich beinhaltet ist und die dargestellt werden soll, definiert sein können.

Voranstehend wurden Techniken beschrieben, bei welchen das Auswählen der mindestens einen Messspule basierend auf dem Messbereich und der Vorauswahl basiert. Im Allgemeinen können alternativ oder zusätzlich auch weitere Entscheidungskriterien im Zusammenhang mit dem Auswählen der mindestens einen Messspule berücksichtigt werden. Beispielsweise wäre es möglich, dass das Verfahren weiterhin umfasst: Empfangen von dritten Steuerdaten, die mindestens eine mit der MR-Anlage verbundene Spule indizieren. Dabei kann das Auswählen der mindestens einen Messspule weiterhin auf der mindestens einen mit der MR-Anlage verbundenen Spule basieren. Dies kann in anderen Worten bedeuten, dass überprüft werden kann, welche der Vielzahl von Kandidaten-Spulen tatsächlich "gesteckt" sind, d.h. mit der MR-Anlage elektronisch verbunden sind.

Das Verfahren kann ferner das Assoziieren des MR-Protokolls mit der mindestens einen Messspule umfassen, nachdem die mindestens eine Messspule ausgewählt wurde. Dies kann in anderen Worten bedeuten, dass keine Vorabassoziierung des MR-Protokolls mit der mindestens einen Messspule existieren muss. Beispielsweise könnte die Assoziation des MR-Protokolls mit der mindestens einen Messspule erst zur Messphase geschehen. Dies macht es entbehrlich, dass eine Vielzahl von unterschiedlichen MR-Protokollen in einer Protokoll-Datenbank vorgehalten wird, wobei die verschiedenen MR-Protokolle alle fix mit Messspulen assoziiert sind. Stattdessen kann es möglich sein, ein und dasselbe MR-Protokoll mit unterschiedlichen Messspulen zu verwenden, je nach der aktuellen Situation, wie sie sich in der Messphase darstellt. Beispielsweise kann dadurch eine abweichende Patientenpositionierung etc. berücksichtigt werden. Es wäre also insbesondere möglich, dass die mindestens eine Messspule nach einer Planungsphase und vor oder während einer Messphase ausgewählt wird. In diesem Zusammenhang wäre es also möglich, dass das MR-Protokoll ohne eine Assoziierung mit der mindestens einen Messspule aus einer Protokoll-Datenbank geladen wird.

Eine MR-Anlage umfasst eine Recheneinheit. Die Recheneinheit ist zur Auswahl von mindestens einer Messspule aus einer Vielzahl von Kandidaten-Spulen der MR-Anlage bei der MR-Bildgebung eingerichtet. Dabei ist die Recheneinheit eingerichtet, um erste Steuerdaten zu empfangen, welche einen Messbereich indizieren. Außerdem ist die Recheneinheit eingerichtet, um zweite Steuerdaten zu empfangen, die eine Vorauswahl aus der Vielzahl von Kandidaten-Spulen indizieren. Ferner ist die Recheneinheit eingerichtet, um basierend auf dem Messbereich und der Vorauswahl mindestens eine Messspule aus der Vielzahl von Kandidaten-Spulen auszuwählen. Außerdem ist die Recheneinheit eingerichtet, um ein MR-Protokoll zu MR-Bildgebung mit der mindestens einen Messspule auszulösen.

Ein Computer-Programm oder ein Computer-Programmprodukt oder ein elektronisch lesbares Speichermedium umfasst Programmcode, der von mindestens einem Prozessor geladen und ausgeführt werden kann. Das Ausführen des Programmcodes bewirkt, dass der mindestens eine Prozessor die folgenden Schritte ausführt: Empfangen von ersten Steuerdaten, die einen Messbereich einer MR-Bildgebung mittels einer MR-Anlage indizieren. Empfangen von zweiten Steuerdaten, die eine Vorauswahl aus einer Vielzahl von Kandidaten-Spulen der MR-Anlage indizieren; Auswählen mindestens einer Messspule aus einer Vielzahl von Kandidaten-Spulen basierend auf dem Messbereich und der Vorauswahl; sowie Auslösen eines MR-Protokolls zur MR-Bildgebung mit der mindestens einen Messspule.

Die oben dargelegten Merkmale sowie Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### KURZE BESCHREIBUNG DER FIGUREN

Figur 1 illustriert schematisch eine MR-Anlage zur MR-Bildgebung;
Figur 2 illustriert schematisch einen Messbereich sowie eine Vorauswahl von Spulen zur MR-Bildgebung
Figur 3 ist ein Flussdiagramm eines verfahrensgemäß verschiedener Beispiele.
Figur 4 illustriert schematisch eine Benutzerschnittstelle zum Treffen einer Vorauswahl von Spulen.

### DETAILLIERTE BESCHREIBUNG VON AUSFÜHRUNGSFORMEN

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionelle Einheiten können aus Hardware, Software oder einer Kombination aus Hardware oder Software implementiert werden.

Nachfolgend werden Techniken zur MR-Bildgebung beschrieben. Insbesondere werden Techniken zum Einstellen von Parametern im Zusammenhang mit der MR-Bildgebung beschrieben. Die hierin beschriebenen Techniken können im Allgemeinen in einer Planungsphase und/oder in bzw. vor einer Messphase durchgeführt werden. Das heißt es wäre zum Beispiel möglich, dass die hierin beschriebenen Techniken in der Planungsphase - wenn eine Untersuchungsperson noch nicht in der MR-Anlage positioniert ist - durchgeführt werden.

Verschiedene hierin beschriebene Techniken ermöglichen es, ein oder mehrere Messspulen zur MR-Bildgebung teilautomatisiert auszuwählen, aus einem Pool von verfügbaren Kandidaten-Spulen. Beispielsweise könnten die Kandidaten-Spulen alle grundsätzlich mit einer MR-Anlage registrierten Spulen indizieren.

Bei der teilautomatisierten Auswahl von ein oder mehreren Messspulen können - beispielsweise neben dem Messbereich und den tatsächlich gesteckten Spulen - auch weitere Parameter berücksichtigt werden. Zum Beispiel wäre es in verschiedenen Beispielen möglich, dass alle im Messbereich befindlichen Spulen ausgewählt werden, jedoch mit Ausnahme von ein oder mehreren nicht-selektierbaren Spulen. Als Beispiel für nicht-selektierbare Spulen könnte zum Beispiel die sogenannte "Wirbelsäulen-Spule" genannt werden, die in Längsrichtung in einem Tisch der MR-Anlage integriert ist. Eine solche Funktionalität kann insbesondere für orthopädische Fragestellungen von Interesse sein, bei denen eine dedizierte Gelenkspule verwendet wird, etwa Knie-Untersuchungen mit einem Knie-Spulenbauteil oder Schulter-Untersuchungen mit einem Schulter-Spulenbauteil oder Hand-Untersuchungen mit einem Hand/Handgelenk-Spulenbauteil oder Sprunggelenkuntersuchungen mit einem Fuß/Knöchel-Spulenbauteil. Außerdem kann eine solche Implementierung für Brust-Untersuchungen erstrebenswert sein, bei welchen die Wirbelsäulen-Spule auf dem Untersuchungstisch verbleibt und ein Brust-Spulenbauteil aufgelegt wird. Wird hier zusätzlich auch die Wirbelsäulen-Spule zusätzlich zu einer oder mehreren Spulen des Brust-Spulenbauteils ausgewählt, so kann die Bildqualität der MR-Bilder aufgrund von Einfaltungen reduziert werden. Dies wird durch das Definieren der Wirbelsäulen-Spule als nicht-selektierbare Spule vermieden.

Aus dem obenstehenden Beispiel ist ersichtlich, dass ein oder mehrere nicht-seletierbaren Spulen als Vorauswahl aus der Vielzahl von Kandidaten-Spulen ausgeschlossen werden können.

In einem weiteren Beispiel wäre es möglich, dass eine Vorauswahl aller möglichen Kandidaten-Spulen durch den Benutzer in positiver Art und Weise getroffen wird. Beispielsweise könnten durch den Benutzer aus einer entsprechenden Auswahlliste Spulenklassen - und ggf. bei bestimmten Spulenklassen, bei denen eine weitere Unterteilung möglich ist, auch sogenannte Spulenkompartments - ausgewählt werden, um derart selektierbare Spulen im Rahmen der Vorauswahl zu definieren. Nachfolgend wird ein Beispiel gegeben, um einen entsprechenden Anwendungsfall zu verdeutlichen: der Nutzer plant eine einseitige Untersuchung der rechten Brust in einer transversalen Bildgebung. Durch einen herkömmlichen Mechanismus zur Auswahl von Spulen gemäß Referenzimplementierungen würden beide Kompartments eines Brust-Spulenbauteils ausgewählt werden, das heißt sowohl ein oder mehrere linke Spulen als auch ein oder mehrere rechte Spulen. Durch die Verwendung der linken und rechten Spulen des Brust-Spulenbauteils käme es zu Einfaltungen und damit zu einer Herabsetzung der Bildqualität. Deshalb wäre es zum Beispiel möglich, dass die entsprechende Vorauswahl das rechte Spulenkompartment des Brust-Spulenbauteils indiziert. Je nach konkret verwendetem Brust-Spulenbauteil können dann ein oder mehrere rechts angeordnete Spulen des Brust-Spulenbauteils ausgewählt werden.

Durch das Berücksichtigen einer solchen Vorauswahl bei der Auswahl von ein oder mehreren Messspulen aus der Vielzahl von Kandidaten-Spulen, kann es entbehrlich werden, dass in den verschiedenen MR-Protokollen eine feste Assoziierung mit ein oder mehreren Spulen erfolgt. Das bedeutet, dass die MR-Protokolle ohne eine Assoziierung mit einer Messspule aus einer Protokoll-Datenbank geladen werden können. Vielmehr kann erst zur Laufzeit die geeignete Messspule oder geeignete MR-Spulen bestimmt werden und eine entsprechende Verknüpfung mit dem ausgewählten MR-Protokoll durchgeführt werden. Dies bedeutet, dass zur Laufzeit, das heißt im Zusammenhang mit der Messphase, die Auswahl einer oder mehrerer Messspule erfolgen kann. Dies ermöglicht es, die MR-Protokolle offline in einer Protokoll-Datenbank ohne Spuleninformation abzulegen und erst zur Laufzeit dynamisch eine optimierte Spuleninformation den jeweiligen MR-Protokollen hinzuzufügen. Damit werden verschiedene Nachteile in Referenzimplementierungen - beispielsweise erst im Zusammenhang mit der Messphase absehbare Parameter der MR-Bildgebung etc. - gelöst. Außerdem kann eine bessere Übertragbarkeit verschiedener MR-Protokolle gewährleistet sein, da die verschiedenen MR-Protokolle nicht mehr aufgrund von Spulen konvertiert werden müssen. Somit können identische MR-Protokolle auch auf verschiedenen MR-Anlagen genutzt werden.

In der Protokollentwicklung kann die Bearbeitungszeit durch die gesteigerte Performance beim Öffnen, Bearbeiten und Speichern von MR-Protokollen verkürzt werden. Konvertierungen aufgrund von geänderten Spuleninformationen werden hinfällig. Die MR-Protokolle sind bei MR-Anlagen mit gleicher Feldstärke, einer ähnlichen Anzahl und Empfangskanälen und gleichen Gradientensystemen austauschbar. Dies könnte zum Beispiel bedeuten, dass die Anzahl von erforderlichen vorkonfigurierten MR-Protokollen in einer Protokoll-Datenbank signifikant reduziert werden kann. Durch die optimale Auswahl von Messspulen zur Laufzeit kann die Notwendigkeit einer Vorauswahl der Messspulen minimiert und die Bildqualität verbessert werden. Ein manuelles Eingreifen in die Auswahl der Messspulen durch den Nutzer, sowie ein Wiederholen von MR-Protokollen wegen eingeschränkter Bildgebung durch falsch selektierte Messspulen wird weniger häufig notwendig sein. Im Zusammenhang mit der Protokollentwicklung können identische MR-Protokolle für unterschiedliche MR-Anlagen verwendet werden, so dass sich die Entwicklungszeit signifikant verkürzt. Das System kann in allen klinisch relevanten Fällen die Spulen auch (teil-)automatisiert bestimmen. Durch eine Effizienzsteigerung bei der Protokollentwicklung und einer drastischen Reduktion der Anzahl der benötigten MR-Protokolle in einer Protokoll-Datenbank kann zukünftig eine Unterstützung aller geplanten Konfigurationen von MR-Anlagen ermöglicht werden. Durch die verbesserte automatisierte Auswahl von Messspulen kann die Bildqualität verbessert werden und weniger manuelle Eingriffe des Benutzers sind möglich. Zudem wird ein Übertragen von MR-Protokollen zwischen verschiedenen MR-Anlagen einfacher, schneller und weniger fehleranfällig möglich.

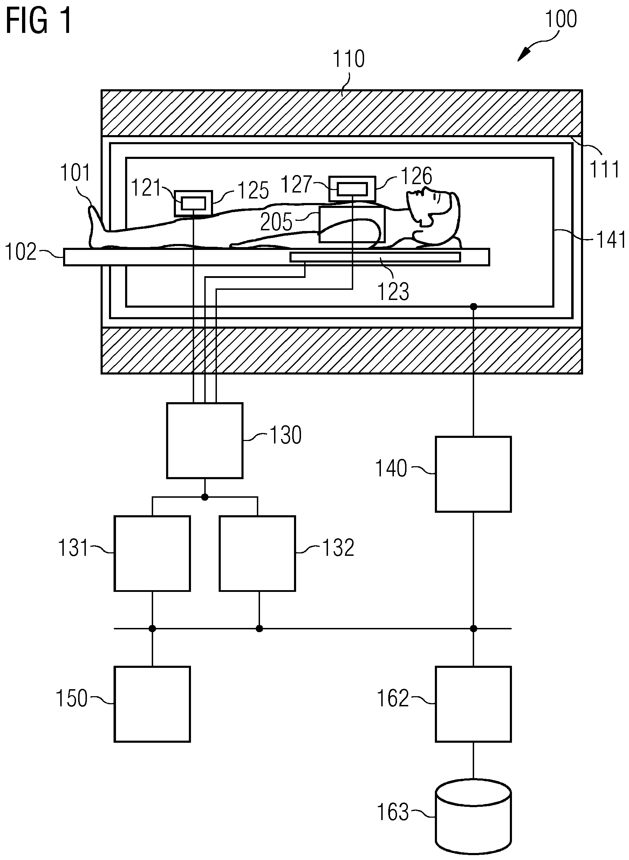

Figur 1 illustriert Aspekte im Zusammenhang mit einer MR-Anlage 100. Die MR-Anlage 100 ist zur MR-Bildgebung eingerichtet. Die MR-Anlage 100 weist einen Magneten 110 auf, der eine Röhre 111 definiert. Der Magnet 110 kann ein Grundmagnetfeld parallel zu seiner Längsachse erzeugen. Ein Untersuchungsobjekt, hier eine Untersuchungsperson 101, ist auf einem Tisch 102 positioniert. Der Tisch 102 kann in den Magneten 110 geschoben werden. Die MR-Anlage 100 weist weiterhin ein Gradientensystem 140 zur Erzeugung von Gradientenfeldern auf, die für die MR-Bildgebung und zur Ortskodierung von erfassten Rohdaten verwendet werden. Dazu sind typischerweise mindestens drei separat ansteuerbare und zueinander wohldefiniert positionierte Gradientenspulen 141 vorgesehen. Die Gradientenspulen 141 ermöglichen es, entlang bestimmter Raumrichtungen (Gradientenachsen) Gradientenfelder anzuwenden und zu schalten. Die Gradientenfelder können zum Beispiel zur Schichtselektion, zur Frequenzkodierung (in Ausleserichtung) und zur Phasenkodierung verwendet werden. Dadurch kann eine Ortskodierung der Rohdaten erreicht werden.

Zur Anregung der sich im Grundmagnetfeld ergebenden Polarisation bzw. Ausrichtung der Kernspins bzw. der Magnetisierung in Längsrichtung werden Sendespulen (in Figur 1 nicht dargestellt) verwendet. Über die Sendespulen ist es möglich, einen amplitudenmodulierten HF-Anregungspuls in einen Messbereich 205 einzustrahlen. Dadurch kann eine Transversalmagnetisierung erzeugt werden.

Zum Empfangen von HF-Signalen der relaxierenden Transversalmagnetisierung werden Empfangsspulen 121-123 verwendet. Dabei können die Empfangsspulen 121-123 in Spulenbauteilen 125-126 wahlweise mit einem HF-Schalter 130 verbunden werden. Beispielsweise ist in dem Beispiel der Figur 1 ein Brust-Spulenbauteil 126 mit einer Brust-Spule 122 dargestellt sowie ein Knie-Spulenbauteil 125 mit einer Knie-Spule 121. Eine Brust-Untersuchung kann auch in Bauchlage durchgeführt werden. Manche Spulen können auch fest mit der MR-Anlage 100 verbaut sein, beispielsweise im Szenario der Figur 1 eine Wirbelsäulen-Spule 123, die sich entlang des Tisches 102 in Richtung Kopf-Fuß der Untersuchungsperson 101 erstreckt. Je nachdem, welche Zielsetzung die MR-Bildgebung erfolgt, können unterschiedliche dieser Empfangsspulen 121-123 mit der MR-Anlage 100 verbunden werden. Dabei können entsprechende Steuerdaten an eine Rechnereinheit 162 übertragen werden, wobei die Steuerdaten indizieren, welche Empfangsspule 121-123 mit dem HF-Schalter 130 verbunden sind, d.h. "gesteckt" sind.

Als allgemeine Regel könnten die Empfangsspulen 121-123 oder zumindest einzelne der Empfangsspulen 121-123 auch als Sendespulen verwendet werden. Deshalb kann der HF-Schalter 130 ausgebildet sein, um die Spulen 121-123 wahlweise mit einer HF-Sendeeinheit 131 oder einer HF-Empfangseinheit 132 zu verbinden. Die HF-Sendeeinheit 131 kann einen HF-Generator in einer HF-Amplitudenmodulationseinheit umfassen. Mittels der HF-Empfangseinheit 132 können HF-Signale der relaxierenden Transversalmagnetisierung durch induktives Einkoppeln in die entsprechenden Empfangsspulen 121-123 erfasst werden.

Während in dem Beispiel von Figur 1 ein Szenario dargestellt ist, in welchem die Spulenbauteile 125-126 jeweils eine einzelne Spule 121-122 umfassen, wäre es in anderen Beispielen auch möglich, dass Spulenbauteile mehrere Spulenkompartments definieren in denen jeweils eine oder mehrere Spulenelemente angeordnet sind.

Außerdem wäre es in verschiedenen Beispielen möglich, dass die Spulen als Array-Spulenelemente ausgeführt sind, was insbesondere im Zusammenhang mit der bildbeschleunigten MR-Bildgebung hilfreich sein kann.

Die MR-Anlage 100 weist weiterhin eine Benutzerschnittstelle 150 auf, welche zum Beispiel einen Bildschirm, eine Tastatur, eine Maus etc. umfassen kann. Mittels der Benutzerschnittstelle 150 kann eine Benutzereingabe erfasst werden und Ausgabe zum Benutzer realisiert werden. Beispielsweise könnte es möglich sein, mittels der Benutzerschnittstelle 150 einzelne Betriebsmodi bzw. Betriebsparameter der MR-Anlage durch den Benutzer und/oder/automatisch und/oder ferngesteuert einzustellen. Zum Beispiel wäre es möglich, mittels der Benutzerschnittstelle 150 den Messbereich 205 festzulegen oder aber auch eine Vorauswahl im Zusammenhang mit der Auswahl von ein oder mehreren Empfangsspulen 121-123 zu treffen.

In dem Beispiel der Figur 1 ist die Recheneinheit 162 weiterhin mit einem Datenbanksystem 163 verbunden, welches zum Beispiel eine Protokoll-Datenbank und/oder eine Spulen-Datenbank implementieren kann. Dabei könnte die Spulen-Datenbank eine Vielzahl von Kandidaten-Spulen, die mit der MR-Anlage 100 kompatibel sind, indizieren. Zum Beispiel könnte das Datenbanksystem 163 in einer Cloud-Lösung implementiert sein.

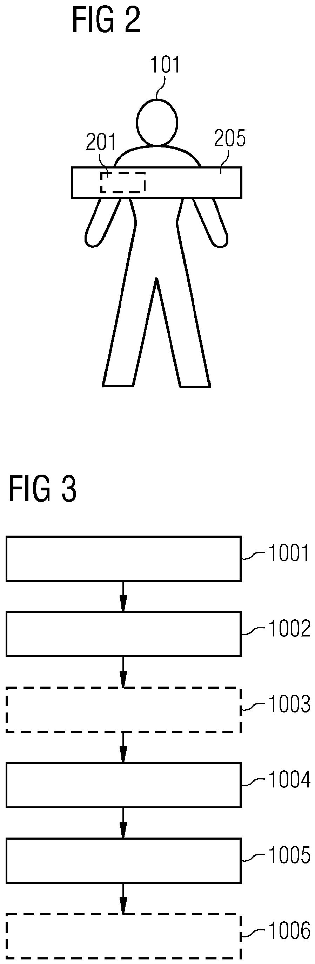

Figur 2 illustriert weiterhin Aspekte im Zusammenhang mit dem Messbereich 205 und einer Vorauswahl 201 betreffend die Vielzahl von Kandidaten-Spulen. Typischerweise kann es eine große Anzahl von möglichen Kandidaten-Spulen geben, die mit der MR-Anlage 100 kompatibel sind. Zum Beispiel kann eine Vielzahl von unterschiedlichen Spulen gesteckt werden, je nach anatomischer Region, die durch den Messbereich 205 definiert ist. Außerdem kann es auch im Zusammenhang mit dem Messbereich 205 möglich sein, unterschiedliche Spulen 121-123 auszuwählen: zum Beispiel könnte mittels der Wirbelsäulen-Spule 123, wie auch mittels der Brust-Spule 122 im Szenario der Figur 1 ein HF-Signal gemessen werden.

Deshalb kann es manchmal erstrebenswert sein, die Vorauswahl 201 im Zusammenhang mit der Vielzahl von Kandidaten-Spulen zu treffen (etwa alle gestreckten Empfangsspulen 121-123, vgl. Figur 1). Dann ist es möglich, dass basierend auf dem Messbereich 205 sowie dieser Vorauswahl 201 mindestens eine Messspule aus der Vielzahl von Kandidaten-Spulen ausgewählt wird. Im Beispiel der Figur 2 betrifft die Vorauswahl ein Spulenkompartment des Brust-Spulenbauteils 126. Insbesondere betrifft die Vorauswahl 201 das linke Spulen-Kompartment des Spulenbauteils 126. Im linken Spulenkompartment befinden sich ein oder mehrere Spulenelemente 122 des Brust-Spulenbauteils 126. Im Allgemeinen wäre es möglich, dass die Vorauswahl 201 ein oder mehrere selektierbare Spulenklassen oder Spulenkompartments definiert. Zum Beispiel könnten die ein oder mehrere Spulenklassen oder Spulenkompartments im Zusammenhang mit einer assoziierten anatomischen Region (im Beispiel der Figur 2 die linke Brust) definiert sein.

In weiteren Beispielen könnte die Vorauswahl aber zum Beispiel auch ein oder mehrere nicht-selektierbare Spulen definieren. Im Zusammenhang mit dem Szenario der Figur 1 könnten die ein oder mehrere nicht-selektierbaren Spulen zum Beispiel die Wirbelsäulen-Spule 123 betreffen.

Im Beispiel der Figur 2 ist ersichtlich, dass die durch die Vorauswahl 201 definierte anatomische Region ("linke Brust") kleiner ist, als die anatomische Region, die durch den Messbereich 205 definiert ist ("Brustbereich"). Dadurch kann erreicht werden, dass die geeignete Spule des Brust-Spulenbauteils 126 ausgewählt wird.

Figur 3 ist ein Flussdiagramm eines beispielhaften Verfahrens. Beispielsweise könnte es möglich sein, dass die Recheneinheit 162 entsprechenden Programmcode aus einem Speicher lädt und anschließend ausführt, um das Verfahren gemäß Figur 3 durchzuführen.

Zunächst werden in Block 1001 erste Steuerdaten empfangen, welche einen Messbereich indizieren. Der Messbereich kann zum Beispiel im Zusammenhang mit einer anatomischen Region definiert sein.

Anschließend werden in Block 1002 zweite Steuerdaten empfangen, welche eine Vorauswahl aus einer Vielzahl von Kandidaten-Spulen indizieren.

Im Allgemeinen wäre es möglich, dass die ersten und zweiten Steuerdaten in den Blöcken 1001, 1002 von einer Benutzerschnittstelle durch eine Benutzereingabe empfangen werden.

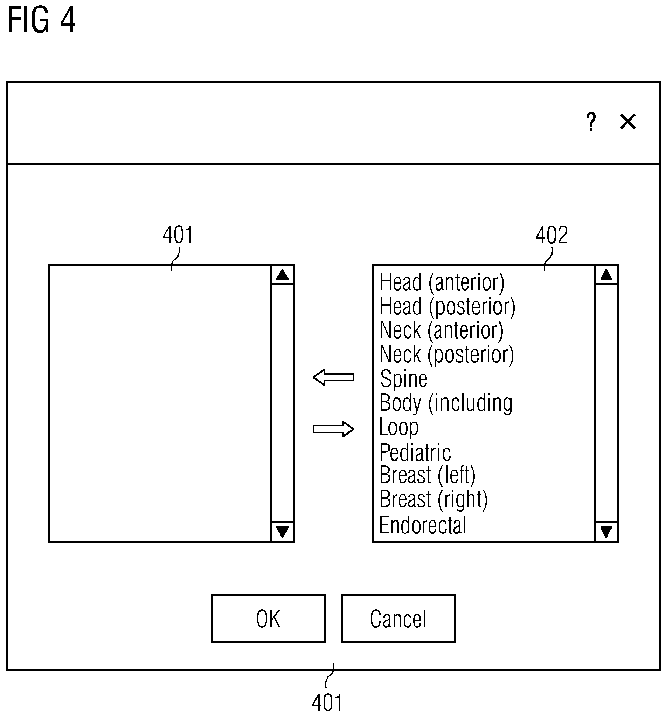

Eine beispielhafte Implementierung für eine Benutzerschnittstelle zum Festlegen der Vorauswahl in Block 1002 ist zum Beispiel in Figur 4 dargestellt. In Figur 4 ist ein Menüdialog 401 dargestellt, der zum Beispiel in einem Steuerprogramm, welches auf einem Bildschirm wiedergegeben werden kann, ausgeführt werden könnte. Dabei gibt es zwei Auswahlfelder 401, 402. Im Auswahlfeld 402 ist eine Liste aller definierbaren Spulenklassen und Spulenkompartments hinterlegt. Einzelne oder mehrere dieser Spulenklassen und Spulenkompartments können anschließend in das Auswahlfeld 401 durch den Benutzer überführt werden, so dass derart die entsprechende Vorauswahl von selektierbaren Spulen - parametrisiert mittels der Spulenklassen und Spulenkompartments - festgelegt werden kann.

Wieder bezugnehmend auf Figur 3: anschließend können im optionalen Block 1003 auch dritte Steuerdaten empfangen werden, welche mindestens eine mit der MR-Anlage verbundene Spule indizieren. Dies bedeutet, dass die dritten Steuerdaten zum Beispiel solche Spulen indizieren können, die elektronisch mit einem entsprechenden HF-Schalter der MR-Anlage durch Herstellen einer entsprechenden Steckverbindung verbunden sind. Anschließend erfolgt in Block 1004 das Auswählen einer oder mehrerer Messspulen. Diese könnte z.B. vor und während der Messphase erfolgen.

Dabei beruht das Auswählen der ein oder mehreren Messspulen in Block 1004 auf den Steuerdaten, die in den Blöcken 1001-1002 und optional Block 1003 empfangen wurden. Dies bedeutet in anderen Worten, dass die ein oder mehreren Messspulen basierend auf dem Messbereich, der Vorauswahl und/oder den aktivierten Spulen ausgewählt werden können. Zum Beispiel können bei der Auswahl solche Spulen ignoriert werden, die als nicht-selektierbare Spulen durch die zweiten Steuerdaten indiziert werden. Es könnten auch solche Spulen ignoriert werden, die nicht als selektierbare Spulen durch die zweiten Steuerdaten in Block 1002 indiziert werden.

Anschließend erfolgt in Block 1005 das Auslösen eines MR-Protokolls. Dazu kann das MR-Protokoll aus einer Protokoll-Datenbank geladen werden.

Beispielsweise könnte ein entsprechender Steuerbefehl an eine Protokoll-Steuereinheit übergeben werden, so dass anschließend die MR-Bildgebung mittels des MR-Protokolls und anhand der ausgewählten ein oder mehreren Messspulen erfolgt.

Das MR-Protokoll könne ohne eine Assoziierung aus der Protokoll-Datenbank geladen werden: Optional kann anschließend in Block 1006 ein Assoziieren des MR-Protokoll aus Block 1005 mit den ein oder mehreren ausgewählten Messspulen aus Block 1004 erfolgen. Dies bedeutet, dass zum Beispiel das MR-Protokoll mit einem Querverweis in einer Protokoll-Datenbank abgelegt werden könnte.

Zusammenfassend wurden voranstehend Techniken beschrieben, bei denen eine teilautomatisierte oder auch vollautomatisierte Auswahl von ein oder mehreren Messspulen erfolgen kann. Beispielsweise können insbesondere ein oder mehrere Empfangsspulen als Messspulen ausgewählt werden. Mittels der voranstehend beschriebenen Techniken können unterschiedliche Probleme von Referenzimplementierungen behoben werden. Beispielsweise kann es möglich sein, MR-Protokolle in einer Protokoll-Datenbank abzulegen, ohne einen festen Querverweis zu ein oder mehreren Messspulen herzustellen. Dies ermöglicht es, MR-Protokolle zwischen verschiedenen MR-Anlagen besonders einfach zu konvertieren, weil die MR-Protokolle nicht mit gegebenenfalls MR-Anlage-spezifischen Spulen verknüpft sind.

Außerdem kann eine besonders optimale Auswahl von Messspulen erreicht werden. Insbesondere können solche Spulen, welche möglicherweise zu Beeinträchtigungen der Bildqualität durch Einfaltungen führen (typischerweise zum Beispiel eine Wirbelsäulen-Spule) als nicht-selektierbare Spulen im Rahmen der Vorauswahl ausgeschlossen werden. Dies kann insbesondere im Zusammenhang mit der Messphase geschehen, so dass keine Notwendigkeit besteht, ein oder mehrere Messspulen in der Planungsphase ohne konkrete Kenntnis der Patientendaten auszuwählen (beispielsweise Patientengröße und Patientenlagerung). Eine nachträgliche Anpassung der MR-Protokolle ist demnach entbehrlich. Eine Anpassung kann z.B. lediglich in dem Fall durchgeführt werden, wenn die Bildgebung nicht die erwünschten Ergebnisse zeigt bzw. die Spulenauswahl fehlschlägt. Eine Konvertierung bei der Übertragung von MR-Protokollen zwischen unterschiedlichen MR-Anlagen, die für die Verwendung unterschiedlicher Spulen konfiguriert sind, kann einfacher erfolgen. Beispielsweise kann eine Konvertierung der MR-Protokolle entfallen, weil keine Vorab-Assoziierung der MR-Protokolle mit einzelnen Spulen erfolgen muss.

Selbstverständlich können die Merkmale der vorher beschriebenen Ausführungsformen und Aspekte der Erfindung miteinander kombiniert werden. Insbesondere können die Merkmale nicht nur in den beschriebenen Kombinationen, sondern auch in anderen Kombinationen oder für sich genommen verwendet werden, ohne das Gebiet der Erfindung zu verlassen.

Beispielsweise wurden voranstehend Techniken beschrieben, bei denen insbesondere ein oder mehrere Empfangsspulen als Messspulen im Zusammenhang mit einer Vorauswahl indiziert werden können. Entsprechende Techniken können auch zum Treffen einer Vorauswahl in Bezug auf eine Sendespule implementiert werden.

## Patentansprüche

1. Verfahren zur Auswahl von mindestens einer Messspule (121-123) aus einer Vielzahl von Kandidaten-Spulen (121-123) einer Magnetresonanz-Anlage (100) bei der Magnetresonanz-Bildgebung, wobei das Verfahren umfasst:
- Empfangen von ersten Steuerdaten, die einen Messbereich (205) indizieren,
- Empfangen von zweiten Steuerdaten, die eine Vorauswahl (201) aus der Vielzahl von Kandidaten-Spulen (121-123) indizieren,
- basierend auf dem Messbereich (205) und der Vorauswahl (201): Auswählen der mindestens einen Messspule (121-123) aus der Vielzahl von Kandidaten-Spulen (121-123), und
- Auslösen eines Magnetresonanz-Protokolls zur Magnetresonanz-Bildgebung mit der mindestens einen Messspule (121-123).

2. Verfahren nach Anspruch 1, wobei die zweiten Steuerdaten ein oder mehrere nicht-selektierbare Spulen im Zusammenhang mit der Vorauswahl (201) indizieren.

3. Verfahren nach Anspruch 2, wobei die ein oder mehreren nicht-selektierbaren Spulen eine Wirbelsäulen-Spule umfassen.

4. Verfahren nach einem der voranstehenden Ansprüche, wobei die zweiten Steuerdaten ein oder mehrere selektierbare Spulenklassen oder Spulenkompartments umfassen.

5. Verfahren nach Anspruch 4, wobei die ein oder mehreren selektierbaren Spulenklassen oder Spulenkompartments im Zusammenhang mit einer assoziierten anatomischen Region definiert sind.

6. Verfahren nach Anspruch 5, wobei der Messbereich (205) im Zusammenhang mit einer weiteren anatomischen Region definiert ist, die größer ist, als die anatomische Region.

7. Verfahren nach einem der voranstehenden Ansprüche, das weiterhin umfasst:
- Empfangen von dritten Steuerdaten, die mindestens eine mit der Magnetresonanz-Anlage (100) verbundene Spule indizieren,
wobei das Auswählen der mindestens einen Messspule (121-123) weiterhin auf der mindestens einen mit der Magnetresonanz-Anlage (100) verbundenen Spule basiert.

8. Verfahren nach einem der voranstehenden Ansprüche, das weiterhin umfasst:
- Assoziieren des Magnetresonanz-Protokolls mit der mindestens einen Messspule (121-123) nachdem die mindestens eine Messspule (121-123) ausgewählt wurde.

9. Verfahren nach einem der voranstehenden Ansprüche, wobei die mindestens eine Messspule (121-123) nach einer Planungsphase und vor oder während einer Messphase ausgewählt wird.

10. Verfahren nach einem der voranstehenden Ansprüche, wobei das Magnetresonanz-Protokoll ohne eine Assoziierung mit der mindestens einen Messspule (121-123) aus einer Protokoll-Datenbank geladen wird.

11. Magnetresonanz-Anlage (100) mit einer Recheneinheit (162), die zur Auswahl von mindestens einer Messspule (121-123) aus einer Vielzahl von Kandidaten-Spulen (121-123) der Magnetresonanz-Anlage (100) bei der Magnetresonanz-Bildgebung eingerichtet ist, wobei die Recheneinheit eingerichtet ist, um folgende Schritte auszuführen:
- Empfangen von ersten Steuerdaten, die einen Messbereich (205) indizieren,
- Empfangen von zweiten Steuerdaten, die eine Vorauswahl (201) aus der Vielzahl von Kandidaten-Spulen (121-123) indizieren,
- basierend auf dem Messbereich (205) und der Vorauswahl (201): Auswählen der mindestens einen Messspule (121-123) aus der Vielzahl von Kandidaten-Spulen (121-123), und
- Auslösen eines Magnetresonanz-Protokolls zur Magnetresonanz-Bildgebung mit der mindestens einen Messspule (121-123).

12. Magnetresonanz-Anlage (100) nach Anspruch 11, wobei die Recheneinheit eingerichtet ist, um das Verfahren nach einem der Ansprüche 1 bis 10 auszuführen.
